# EUROPEAN PATENT APPLICATION

(11) **EP 0 650 725 A1**
(43) Date of publication of application: **03.05.1995**
(21) Application number: 94308004.4
(22) Date of filing: 31.10.1994
(51) Int. Cl.: A61K 31/425

(54) **Compositions containing salts of L-2-oxothiazolidine-4-carboxylic acid and their use for stimulating intracellular glutathione**

(30) Priority: 03.11.1993 US 147165
(71) Applicant: FREE RADICAL SCIENCES Inc, Cambridge Massachusetts 02139 (US)
(72) Inventor: Pace, Gary, Cambridge, MA 02142 (US)
(74) Representative: MacGregor, Gordon

(57) **Abstract**

The composition comprises salt of L-2-oxothiazolidine-4-carboxylic acid. The salt may include at least one cation chosen from sodium; potassium; magnesium, calcium, iron; zinc; copper; calcium; selenium; manganese; and molybdenum for administration of the cation to a patient, for example, for treatment of osteoporosis, malnutrition, dermatological dysfunction, or glucose intolerance.

## Description

### BACKGROUND OF THE INVENTION

The present invention relates generally to the treatment of disease states. More specifically, the present invention relates to compositions and methods for stimulating intracellular glutathione levels.

It is known that intracellular levels of glutathione can be important with respect to cell function and the overall well being of mammals. Reduced glutathione levels are found in many disease states, e.g., immune compromised patients and stressed patients.

Additionally, it is known that glutathione provides many benefits in protecting cells against damage. For example, glutathione protects cells against the effects of free radicals and oxygen intermediates. Free radicals are molecules with an unpaired electron creating an unstable and highly reactive molecule. Oxygen free radicals are highly reactive with biological macromolecules, such as found in cell membranes and thereby can induce cell damage.

A number of methods of treatment have been devised using the stimulation of intracellular glutathione levels. Such treatments have been proposed for a variety of disease states, including: reperfusion injury (see U.S. Patent No. 5,095,027); hepatic disease; adult respiratory distress syndrome; immune disorders; bone marrow hypoplasia; acquired immune deficiency syndrome; infertility; atherosclerosis; and latent viral infections.

Unfortunately, according to literature in the art, intracellular glutathione levels cannot be increased by merely attempting to load the cell with glutathione. See, for example, U.S. Patent No. 4,784,685 - "there are several reports on particular biological systems indicating that glutathione itself is not transported into cells" (column 2, lines 37-39).

Glutathione is composed of three amino acids: glutamic acid; cysteine; and glycine. Although the administration to animals of amino acid precursors of glutathione may produce an increase in cellular glutathione, there is a limit to the effectiveness of this procedure.

Concentrations of glutathione are dependent on the supply of cysteine. Cysteine can be derived from dietary protein and by transulfuration from methionine in the liver. Cysteine administration is not an ideal method for increasing intracellular glutathione concentrations. In this regard, cysteine is rapidly metabolized and is very toxic. See, for example, U.S. Patent No. 4,434,158 - "cysteine cannot be administered intravenously due to its toxic effects on the system" (column 2, lines 6-8).

A number of compounds have been devised for increasing intracellular glutathione levels. For example, it is known to administer N-acetyl-L-cysteine, L-2-oxothiazolidine-4-carboxylic acid, and glutathione esters. Examples of patents relating to L-2-oxothiazolidine-4-carboxylic acid and glutathione esters are as follows: 4,335,210; 4,434,158; 4,438,124; 4,647,751; 4,665,082; 4,710,489; and 4,784,685. It should be noted that in U.S. Patent Nos. 4,335,210; 4,434,158; 4,458,124; 4,647,751; and 4,665,082 that the acid L-2-oxothiazolidine-4-carboxylic acid is referred to as L-2-oxothiazolidine-4-carboxylate. A number of other patent applications and patents have adopted this nomenclature. Applicant believes that the more proper nomenclature is L-2-oxothiazolidine-4-carboxylic acid as only the acid form has been described or disclosed by any of the patents or applications.

L-2-oxothiazolidine-4-carboxylic acid is transported into most cells where it is converted by the action of 5-oxo-L-prolinase in the presence of adenosine triphosphate to produce S-carboxyl cysteine. S-carboxyl cysteine is then decarboxylated to produce cysteine. Cysteine is then rapidly used for glutathione synthesis.

Esters of L-2-oxothiazolidine-4-carboxylic acid have recently been disclosed. See, U.S. Patent No. 5,208,249 which provides a method and composition for stimulating the intracellular synthesis of glutathione through the administration of a therapeutically effective amount of an ester of 2-oxothiazolidine-4-carboxylic acid. The ester can be a saturated straight or branched, alkyl group of 1 to 10 carbon atoms.

L-2-oxothiazolidine-4-carboxylic acid is believed to have many therapeutic effects. At the present time, L-2-oxothiazolidine-4-carboxylic acid is being explored in FDA approved clinical trials as a possible therapeutic agent which can be used in the treatment of acquired immune deficiency syndrome.

L-2-oxothiazolidine-4-carboxylic acid is produced as an acid. The pH of L-2-oxothiazolidine-4-carboxylic acid is approximately 2. When L-2-oxothiazolidine-4-carboxylic acid is administered orally, it is prepared as a capsule or tablet. Due to the acidic nature of L-2-oxothiazolidine-4-carboxylic acid, several problems could be caused by the oral form of L-2-oxothiazolidine-4-carboxylic acid. Perhaps, the chief issue caused by the acidic nature of L-2-oxothiazolidine-4-carboxylic acid is with respect to gastrointestinal problems in patients. Due to its acidic nature, L-2-oxothiazolidine-4-carboxylic acid can cause acid stomach upset. This can be a significant side effect in patients with stomach ulcers which can be a common syndrome exhibited by patients with acquired immune deficiency syndrome.

The acidic form of L-2-oxothiazolidine-4-carboxylic acid can also create issues with respect to formulating the product. As a parenteral product, L-2-oxothiazolidine-4-carboxylic acid must be neutralized or the product cannot be administered intravenously. The neutralization step increases the cost of production. Still further, the low pH of L-2-oxothiazolidine-4-carboxylic acid can cause an acceleration in degregation of other ingredients that are co-administered or formulated with L-2-oxothiazolidine-4-carboxylic acid.

A further, contradictory issue with respect to L-2-oxothiazolidine-4-carboxylic acid is that when neutralized, it is difficult to lyophilize. Lyophilization of L-2-oxothiazolidine-4-carboxylic acid may create a variety of useful products. See, for example, U.S. patent application Serial Nos. 08/010,539 and 08/010,653, entitled: "LYOPHILIZED ENTERALLY ADMINISTRABLE L-2-OXOTHIAZOLIDINE-4-CARBOXYLATE" and "LYOPHILIZED PARENTERALLY ADMINISTRABLE L-2-OXOTHIAZOLIDINE-4-CARBOXYLATE", respectively.

### SUMMARY OF THE INVENTION

The present invention provides salts of L-2-oxothiazolidine-4-carboxylic acid, as well as methods of using same. These salts can be utilized to stimulate intracellular glutathione synthesis without the need for neutralization or without causing gastrointestinal concerns in certain patients. Moreover, by selecting the appropriate salt, a specific cation can be delivered to the patient thereby supplementing in the patient the required cation or adjusting the load or ratio of another cation. Also, if desired, the salt can be combined with the acid form of 1-2-oxothiazolidine-4-carboxylate to give a resulting formulation of differing pHs.

To this end, the present invention provides a method for stimulating the intracellular synthesis of glutathione comprising the step of administering to a mammal a therapeutically effective amount of a salt of L-2-oxothiazolidine-4-carboxylic acid.

In an embodiment, the salt includes a cation chosen from the group consisting of: sodium; potassium; magnesium; calcium; iron; zinc; copper; calcium; selenium; manganese; and molybdenum.

In an embodiment of the method, at least two different salts of L-2-oxothiazolidine-4-carboxylic acid are administered.

In an embodiment, the method includes the step of administering the salt of L-2-oxothiazolidine-4-carboxylic acid in combination with L-2-oxothiazolidine-4-carboxylic acid.

In an embodiment, the salt of L-2-oxothiazolidine-4-carboxylic acid is administered enterally.

In an embodiment, the salt of L-2-oxothiazolidine-4-carboxylic acid is administered parenterally.

In an embodiment, the salt of L-2-oxothiazolidine-4-carboxylic acid is administered topically.

The present invention also provides a composition for stimulating intracellular glutathione synthesis comprising: 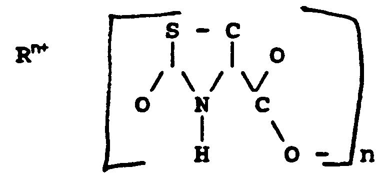
wherein R is a cation and n is a whole number.

In an embodiment, R is chosen from the group consisting of: sodium; potassium; magnesium; calcium; iron; zinc; copper; calcium; selenium; manganese; and molybdenum.

In an embodiment, the present invention provides a method for treating specific disease states that benefit from increased levels of intracellular glutathione comprising the steps of administering a salt of L-2-oxothiazolidine-4-carboxylic acid to a patient having such a disease state and choosing the salt of L-2-oxothiazolidine-4-carboxylic acid so as to simultaneously deliver to the patient a desirable cation.

It is an advantage of the present invention to provide a composition for stimulating intracellular glutathione levels.

Still further, an advantage of the present invention is to provide a composition for stimulating intracellular glutathione levels which can also provide at the same time a specific cation to the patient.

Moreover, an advantage of the present invention is to provide a composition for stimulating intracellular glutathione levels that does not cause gastrointestinal issues.

A further advantage of the present invention is to provide a composition for stimulating intracellular glutathione levels that is easily lyophilized.

Additionally, an advantage of the present invention is to provide a method for delivering specific cation(s) to a patient.

Furthermore, an advantage of the present invention is to provide a composition that can be tailored to treat specific disease states.

Another advantage of the present invention is to provide a composition for stimulating intracellular glutathione levels that may be easier to manufacture on a commercial level than L-2-oxothiazolidine-4-carboxylic acid.

Still, an advantage of the present invention is to provide a composition for stimulating intracellular glutathione levels that can be administered parenterally, enterally, or topically.

Additional features and advantages of the present invention are described in, and will be apparent from, the detailed description of the presently preferred embodiments.

### DETAILED DESCRIPTION OF THE PRESENTLY PREFERRED EMBODIMENTS

The present invention provides methods and compositions for increasing the intracellular synthesis of glutathione. Pursuant to the method of the present invention, a therapeutically effective amount of a salt of L-2-oxothiazolidine-4-carboxylic acid is administered to a patient.

It has been found that the salt can be used to increase the intracellular synthesis of glutathione. Still further, by selecting the appropriate salt, specific cations can be delivered to the patient allowing not only the stimulation of intracellular glutathione synthesis to be used to treat the patient, but the cations as well can be used to assist in treatment.

Preferably, the salt has the structure: 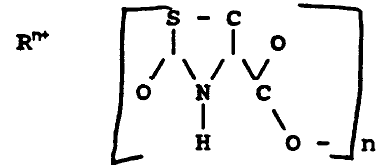
wherein R is a cation and n is a whole number.

A variety of salts can be utilized depending on the specific patient to be treated and/or the specific result desired. For example, R can be chosen from the group consisting of: sodium; potassium; magnesium; calcium; iron; zinc; copper; chromium; selenium; manganese; and molybdenum.

Depending on the specific disease state or rationale for treatment, one or more salts of L-2-oxothiazolidine-4-carboxylic acid may be desirable.

With respect to sodium, sodium is desirable from the standpoint that it has a reduced cost and therefore can provide a salt of L-2-oxothiazolidine-4-carboxylic acid at reduced cost. Moreover, sodium can be used in most cases wherein the purpose of the salt of L-2-oxothiazolidine-4-carboxylic acid is to merely stimulate intracellular glutathione synthesis. However, in certain instances, it may not be desirable to use the salt of sodium, for example, where one is treating a cardiac patient or severely malnourished patient and is concerned about sodium overload.

Potassium is a desirable cation to use especially with respect to patients who suffer from malnutrition including clinical surgical malnutrition. Potassium is the main intracellular cation. Potassium deficiency is an integral part of malnutrition and needs repletion apart from nitrogen. Potassium supplementation will also be desirable in patients suffering alcoholic liver disease.

After potassium, magnesium is the most abundant intracellular cation. Intracellularly magnesium is bound to protein and it is necessary for certain vital functions including atpase and adenylate cyclase and the synthesis of nuclear DNA. It is also necessary for the control of neuromuscular excitability. Magnesium is also required for patients suffering from malnutrition. Further, magnesium may be required for short bowel disease, diarrhea and alcohol liver diseases.

Calcium can be utilized as the cation especially with patients who require growth or who suffer from osteoporosis. Additionally, calcium can be used to control phosphate elevation in renal disease patients.

R can also include micronutrients and specifically trace elements.

Iron can be supplied through the use of a salt of L-2-oxothiazolidine-4-carboxylic acid. By using a salt of L-2-oxothiazolidine-4-carboxylic acid that includes iron, it provides a bioavailable iron source. This may be required in certain patients suffering from anemia as well as certain TPN patients and malnourished patients.

Zinc may be desirable for certain dermatological applications. U.S. Patent No. 5,208,249, the disclosure of which is incorporated herein by reference, discloses esters of L-2-oxothiazolidine-4-carboxylic acid. It may be desirable to use the ester of L-2-oxothiazolidine-4-carboxylic acid with a salt of L-2-oxothiazolidine-4-carboxylic acid based on zinc.

In addition to dermatological benefits, zinc provides advantages with respect to cellular immunity, growth, fertility, and alcoholic liver disease. Zinc deficiencies can alter protein synthesis at a number of different points. In the absence of zinc, growth arrest occurs. Indeed, zinc deficiency may effect gene expression.

Copper is required for oxidative enzymes, e.g., superoxide dismutase activity. Additionally, copper is required for collagen formation. The use of copper to create a salt of L-2-oxothiazolidine-4-carboxylic acid may be desirable in a number of indications including leukopenia.

The chromium salt of L-2-oxothiazolidine-4-carboxylic acid would be desirable especially with respect to diabetes and acute illness. Chromium is distributed throughout the body, however, its concentration decreases with age. It has been found that a decline in body chromium may contribute to the glucose intolerance in the elderly.

Selenium is required for glutathione peroxidase activity. Additionally, the selenium salt of L-2-oxothiazolidine-4-carboxylic acid may be desirable in those cases of cardiomyopathy as well as patients suffering reduced Vitamin E requirements.

Manganese is required for mitochondrial superoxide dismutase activity. Manganese is important for the action of glycosyltransferases. A deficiency of manganese may lead to abnormality of cartilage growth in the young. Additionally, manganese is believed to be important for the action of Vitamin K. In certain circumstances, it may be desirable to administer a salt of L-2-oxothiazolidine-4-carboxylic acid based on manganese.

Molybdenum is required for oxidative metabolism. It is an essential component of xanthine oxidase, sulfite oxidase, and aldehyde oxidase. Molybdenum may be desirable with respect to patients suffering from Chron's disease.

Of course, more than one salt may be administered to a patient to deliver the desirable cations.

There are several processes which can be used to produce salts of L-2-oxothiazolidine-4-carboxylic acid. Depending on the specific salt to be produced, one or more of the processes may be desirable.

### EXAMPLE NO. 1

The simplest process to produce the desired salt is to neutralize L-2-oxothiazolidine-4-carboxylic acid with the appropriate oxide or hydroxide (with the exception of selenium). In this case, the desired oxide or hydroxide (either solid or in solution) is combined in a vessel with a solution of L-2-oxothiazolidine-4-carboxylic acid. The neutralization reaction is carried to completion with stoichiometric amounts of each reactant.

The resulting solution (or slurry) is then concentrated and the appropriate salt of L-2-oxothiazolidine-4-carboxylic acid is crystallized. This crystallized salt is then dried and sieved for the correct size if necessary. The crystallized salt can then be provided as a product in this form.

### EXAMPLE NO. 2

Another process for the manufacture of the desired salt is to use an ion exchange resin, specifically a strong base anion exchange resin. In this process, the anion exchange resin would be put in the hydroxide form. A solution of L-2-oxothiazolidine-4-carboxylic acid would be passed through the resin exchanging the hydroxide ion on the resin for the L-2-oxothiazolidine-4-carboxylic acid ion. The resin is then washed with clean water. Then a chloride or nitrate salt of the desired cation is passed over the resin exchanging the chloride or nitrate in the solution for the L-2-oxothiazolidine-4-carboxylate on the resin.

The desired cation leaves the ion exchanger associated with L-2-oxothiazolidine-4-carboxylic acid, i.e., the desired salt of L-2-oxothiazolidine-4-carboxylic acid. Then the solution of this salt is concentrated and crystallized to make the desired product. The resin can be returned to the hydroxide state by contacting it with an appropriate base, e.g., sodium hydroxide and washing the resin with water.

### EXAMPLE NO. 3

The ion exchange process can be simplified and used to improve the process for the production of some of the more common salts of L-2-oxothiazolidine-4-carboxylic acid, e.g., sodium and potassium. In this case, a solution of L-2-oxothiazolidine-4-carboxylic acid containing other impurities is passed through an anion exchange resin in the hydroxide form. The L-2-oxothiazolidine-4-carboxylic acid attaches itself to the resin whereas the impurities do not. Water then washes the impurities out of the resin. The solution leaving the resin bed in then the potassium salt of L-2-oxothiazolidine-4-carboxylic acid. This potassium salt of L-2-oxothiazolidine-4-carboxylic acid can then be crystallized from this solution, dried, and sieved as before.

### EXAMPLE NO. 4

Preparation of divalent cation salts of L-2-oxothiazolidine-4-carboxylic acid - Preparation of the manganese salt of L-2-oxothiazolidine-4-carboxylic acid.

The less soluble salts of L-2-oxothiazolidine-4-carboxylic acid with divalent cations such as manganese, magnesium and calcium may be prepared by titration of a solution of L-2-oxothiazolidine-4-carboxylic acid with a simple salt of the cation.

A solution containing one mole of L-2-oxothiazolidine-4-carboxylic acid (149 grams) in about 20 ml of water is carefully treated with a concentrated solution of manganese chloride to add a quantity of manganese equivalent to 0.5 moles of the manganese cation. The manganese salt of L-2-oxothiazolidine-4-carboxylic acid (containing two moles of L-2-oxothiazolidine-4-carboxylic acid to each mole of manganese) spontaneously precipitates from solution when held at a temperature below 30 degrees. The precipitated salt of Mn₂L-2-oxothiazolidine-4-carboxylic acid may be collected by filtration or other appropriate method.

### EXAMPLE NO. 5

Preparation of readily soluble monovalent cation salts of L-2-oxothiazolidine-4-carboxylic acid Preparation of the potassium salt of L-2-oxothiazolidine-4-carboxylic acid.

Simple, water soluble monovalent cation salts of L-2-oxothiazolidine-4-carboxylic acid may be efficiently prepared by passing the free acid form of L-2-oxothiazolidine-4-carboxylic acid through a cation exchange column. For preparation of the potassium salt, the solution of L-2-oxothiazolidine-4-carboxylic acid is passed over a cation ion-exchange resin prepared as the potassium salt of the resin.

The cation exchange resin, preferentially prepared as a column for rapid flow of solutions, should contain at a minimum a ten fold excess of exchangeable ions. The solution of L-2-oxothiazolidine-4-carboxylic acid (free acid) is applied to the column, then the column is eluted with at least two column volumes of de-ionized water. The combined eluted solution may then be treated with a non-protic solvent such as acetone or diethyl ether to precipitate the potassium salt of L-2-oxothiazolidine-4-carboxylic acid which may be harvested by filtration or other appropriate method.

The salts of L-2-oxothiazolidine-4-carboxylic acid can be used to create compositions that can be administered enterally, topically, or parenterally.

As a parenteral composition, the salt can be dissolved in standard parenteral solutions. For example, a 3% solution can be provided by adding the appropriate amount of the salt to saline and water for injection.

As an enteral composition, the salt can comprise a portion of a liquid composition that is enterally ingested. For example, the salt can be added to standard complete nutrition enteral formulas or to specialized formulas for specific patients.

Alternatively, the salt can be incorporated in a tablet or capsule with the desired excipients and taken orally by the patient. Of course, as a tablet such excipients can include: starch; lactose; polyvidone; microcrystalline cellulose; magnesium stearate; ethylcellulose; etc. Additionally, if desired, the salt of L-2-oxothiazolidine-4-carboxylic acid can be placed in our oral solution including, for example, sorbitol, glycerol, flavor, and water.

As a topical composition, the salt can be part of a cream or ointment with other necessary components such as paraffin.

The salt of L-2-oxothiazolidine-4-carboxylic acid can be administered alone or in combination with other medicaments. Additionally, if desired, the salt of L-2-oxothiazolidine-4-carboxylic acid can be used in a composition in combination with L-2-oxothiazolidine-4-carboxylic acid. By varying the amounts of each component, the pH can be adjusted to a desired pH. For example, a composition that has a physiologically acceptable pH can be created.

The salts of L-2-oxothiazolidine-4-carboxylic acid can be administered to treat specific diseases that benefit from the stimulation of the intracellular synthesis of glutathione. Such disease states and methods of treatments include those disclosed in U.S. Patent Nos. 5,214,062 and 5,095,027, as well as U.S. Patent Application Nos.: 650,222, entitled: "Method for Insuring Adequate Intracellular Glutathione in Tissue", filed February 2, 1991; 682,636, entitled: "A Method to Enhance Cellular Defense Against Infection", filed April 8, 1991; 769,194, entitled: "Method of Treatment for Latent Virus Infection", filed September 30, 1991; 872,549, entitled: "Method for Reducing or Preventing Toxicity Associated With Antiretroviral Therapy", filed April 23, 1992; 930,183, entitled: "Method for Preventing and Treating Atherosclerosis", filed August 17, 1992; 969,412, entitled: "Method for Optimizing Defensin Synthesis In Vivo and In Vitro", filed October 30, 1992; 971,949, entitled: "Composition and Method for Reducing the Risk of Hypotension", filed November 5, 1992; 983,414, entitled: "Method for Treating Systemic Inflammatory Response Syndrome", filed November 30, 1992; 011,790, entitled: "Method and Composition for Treating Immune Disorders, Inflammation and Chronic Infections", filed February 1, 1993; 012,006, entitled: "Method for Treating Infertility", filed February 1, 1993; 057,122, entitled: "Method for Treatment for Pulmonary Disease", filed May 3, 1993; 059,775, entitled: "Method for Treating Acquired Immune Deficiency Syndrome", filed May 10, 1993; and 068,385, entitled: "Method of Reducing or Preventing Bone Marrow Hypoplasia", filed May 28, 1993, the disclosures of all of these patents and patent applications are incorporated herein by reference.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. Use in a therapeutic preparation of a salt of L-2-oxothiazolidine-4-carboxylic acid for the purpose of stimulating the intracellular synthesis of glutathione in a mammal.

2. Use in a therapeutic preparation for stimulating the intracellular synthesis of glutathione in a mammal, of a salt of L-2-oxothiazolidine-4-carboxylic acid for the additional purpose of therapeutic administration of a specific cation to the mammal.

3. Use according to Claim 2 wherein the salt of L-2-oxothiazolidine-4-carboxylic acid includes at least one cation chosen from: sodium; potassium; magnesium, calcium; iron; zinc, copper; chromium; calcium; selenium; manganese; and molybdenum.

4. Use in a therapeutic preparation of a salt of L-2-oxothiazolidine-4-carboxylic acid, including a cation of potassium, magnesium, or iron, for the purpose of treating a patient suffering from malnutrition.

5. Use in a therapeutic preparation of a calcium salt of L-2-oxothiazolidine-4-carboxylic acid, for the purpose of treating a patient suffering from osteoporosis.

6. Use in a therapeutic preparation of a zinc salt of L-2-oxothiazolidine-4-carboxylic acid, for the purpose of treating a patient suffering from a dermatological dysfunction.

7. Use in a therapeutic preparation of a chromium salt of L-2-oxothiazolidine-4-carboxylic acid, for the purpose of treating a patient suffering from glucose intolerance.

8. Use according to any preceding claim, wherein the preparation includes L-2-oxothiazolidine-4-carboxylic acid so as to provide a composition having a physiologically acceptable pH.

9. Use according to any preceding claim, wherein the preparation is enterally administrable.

10. Use according to any one of Claims 1 to 8, wherein the preparation is parenterally administrable.

11. Use according to any one of Claims 1 to 8, wherein the preparation is topically administrable.

12. Use according to Claim 1 or 2, wherein the preparation includes at least two different salts of L-2-oxothiazolidine-4-carboxylic acid.

13. A composition for assisting in the intracellular synthesis of glutathione and providing a specific cation comprising: 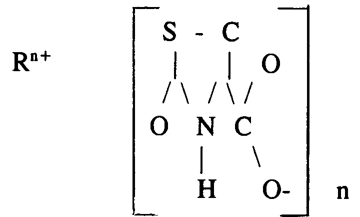 wherein R is a cation chosen from the group consisting of: iron; zinc, copper; calcium; selenium; manganese; chromium; and molybdenum and n is a whole number.
